(19) Europäisches Patentamt
European Patent Office
Office européen des brevets

(11) Veröffentlichungsnummer: **0 507 732 B1**

# (12) EUROPÄISCHE PATENTSCHRIFT

(45) Veröffentlichungstag der Patentschrift: **26.04.95**

(21) Anmeldenummer: **92810214.4**

(22) Anmeldetag: **24.03.92**

(51) Int. Cl.6: **C07C 309/11**, C07C 309/51, D06P 1/649, D06P 3/24

## (54) Asymmetrisches Oxalsäure-diarylamid.

(30) Priorität: **03.04.91 CH 987/91**
**05.02.92 CH 325/92**

(43) Veröffentlichungstag der Anmeldung:
**07.10.92 Patentblatt 92/41**

(45) Bekanntmachung des Hinweises auf die Patenterteilung:
**26.04.95 Patentblatt 95/17**

(84) Benannte Vertragsstaaten:
**AT BE CH DE DK ES FR GB GR IT LI LU NL PT SE**

(56) Entgegenhaltungen:
**US-A- 3 529 982**
**US-A- 3 542 573**

(73) Patentinhaber: **CIBA-GEIGY AG**
**Klybeckstrasse 141**
**CH-4002 Basel (CH)**

(72) Erfinder: **Kaschig, Jürgen, Dr.**
**Rötebuckweg 30**
**W-7800 Freiburg (DE)**
Erfinder: **Reinert, Gerhard, Dr.**
**Weiherweg 1/7**
**CH-4123 Allschwil (CH)**
Erfinder: **Metzger, Georges**
**Herrenweg 228**
**F-68480 Moernach (FR)**

**EP 0 507 732 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues, wasserlösliches, asymmetrisches Oxalsäurediarylamid sowie seine Verwendung zur photochemischen und thermischen Stabilisierung von natürlichen und synthetischen Polyamidfasern und deren Färbungen.

Sulfogruppenhaltige Oxalsäure-diarylamide sind z.B. aus den US-Patentschriften 3 529 982, 4 003 875 und 3 542 573 bekannt. In den ersten beiden Referenzen werden asymmertrische, in der dritten Referenz symmetrische Oxalsäure-diarylamide offenbart. Diese Verbindungen haben jedoch den Nachteil, dass sie gegenüber Polyamidfasern schlechtere Ausziehgrade aufweisen.

Überraschenderweise wurde nun gefunden, dass sich durch geeignete Wahl von sulfogruppenhaltigen Substituenten ein asymmetrisches Oxalsäure-diarylamid herstellen lässt, das wasserlöslich und faseraffin ist. Es eignet sich daher für den Einsatz als photochemischer Stabilisator von natürlichen und synthetischen Polyamidfasern und deren Färbungen und kann in allen üblichen Färbe- und Nachbehandlungsverfahren verwendet werden, wobei mit dieser Verbindung gute Nassechtheiten erzielt werden.

Gegenstand der vorliegenden Erfindung ist demnach ein wasserlösliches, asymmetrisches Oxalsäurediarylamid der allgemeinen Formel

(1) 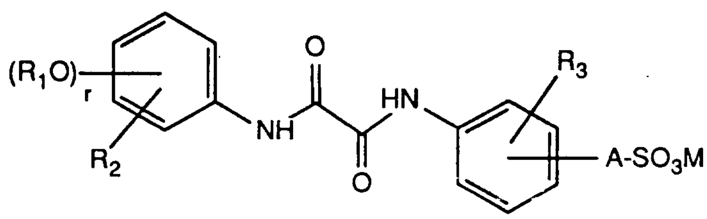 ,

worin

- $R_1$ unabhängig voneinander unsubstituiertes oder durch Hydroxy oder Alkoxy substituiertes $C_1$-$C_5$Alkyl, unsubstituiertes oder durch $C_1$-$C_5$Alkyl substituiertes Benzyl
- $R_2$ Wasserstoff, Halogen, $C_1$-$C_{12}$Alkyl oder Phenyl-$C_1$-$C_5$alkyl
- $R_3$ Wasserstoff, Halogen, $C_1$-$C_{12}$Alkyl, Phenyl-$C_1$-$C_5$alkyl oder $C_1$-$C_5$Alkoxy
- A die direkte Bindung oder einen zweiwertigen Rest der Formel -O-Q-, worin
- Q unsubstituiertes oder durch Hydroxy substituiertes $C_1$-$C_6$Alkylen,
- M Wasserstoff oder Alkalimetall und
- r 2, 1 oder 0 bedeuten.

Bei der Definition der Reste $R_1$ bis $R_3$ stellen $C_1$-$C_5$Alkyl und $C_1$-$C_5$Alkoxy solche Gruppen oder Gruppenbestandteile dar, die 1 bis 5, insbesondere 1 bis 3 Kohlenstoffatome aufweisen. Beispiele für derartige Gruppen sind: Methyl, Ethyl, n-Propyl, Isopropyl, n-Butyl, sek.Butyl, tert.Butyl, Amyl oder Isoamyl bzw. Methoxy, Ethoxy, Isopropoxy, Isobutoxy, tert.-Butoxy oder tert.-Amyloxy.

$C_1$-$C_{12}$Alkyl für die Definition von $R_2$ und $R_3$ können verzweigte oder unverzweigte Reste sein. Es kommen beispielsweise die unter $C_1$-$C_5$Alkyl definierten Vertreter als auch Alkylreste mit höherer Kohlenstoffanzahl in Betracht, wie z.B. Pentyl, Neopentyl, tert.Pentyl, Hexyl, Isohexyl, Heptyl, Ocryl, iso-Octyl, Nonyl, Decyl, Undecyl oder Dodecyl.

$C_1$-$C_6$Alkylen bei der Definition von Q bedeutet einen zweiwertigen, gesättigten Kohlenwasserstoffrest wie z.B. Methylen, Ethylen, Propylen, Trimethylen, Tetrametylen, Ethylethylen, Pentamethylen oder Hexamethylen.

Phenyl-$C_1$-$C_5$Alkyl bedeutet beispielsweise Phenethyl, Phenylpropyl, Phenylbutyl oder vorzugsweise Benzyl.

Halogen bedeutet Fluor, Brom oder Chlor. Chlor ist bevorzugt.

Als Beispiele für Alkalimetalle seien Lithium, Natrium oder Kalium genannt. Bevorzugt ist Natrium.

Vorzugsweise kommt eine Verbindung der Formel (1) in Betracht, bei der Q Trimethylen oder

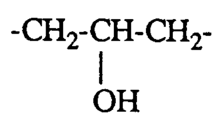

bedeutet.

Ein weiteres bevorzugtes Oxalsäurediarylamid entspricht der Formel

(2) 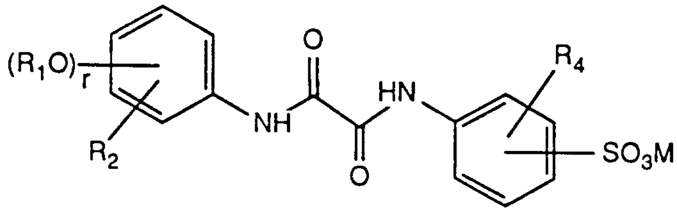 ,

worin

R4 $C_1$-$C_{12}$Alkyl oder $C_1$-$C_5$Alkoxy bedeutet und

$R_1$, $R_2$, M und r die in Formel 1 angegebene Bedeutung haben.

Im Vordergrund des Interesses steht eine Verbindung der Formel

(3) 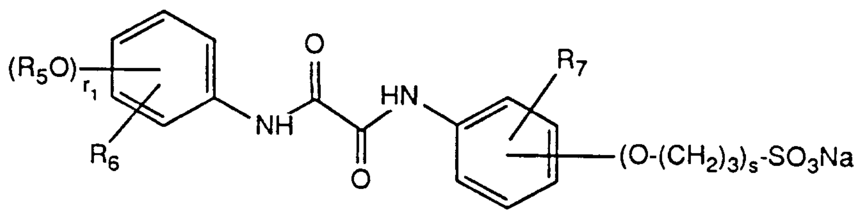 ,

worin

- $R_5$ $C_1$-$C_3$Alkyl,
- $R_6$ Wasserstoff oder $C_1$-$C_3$Alkyl,
- $R_7$ Wasserstoff oder $C_1$-$C_3$Alkoxy
- $r_1$ 0, 1 oder 2, und
- s 0 oder 1 bedeuten.

Ganz besonders bevorzugt ist dabei eine Verbindung, bei der

- $R_5$ Ethyl und
- $R_6$ Wasserstoff oder Ethyl bedeuten.

Hervorzuheben ist die Verbindung der Formel

(4) 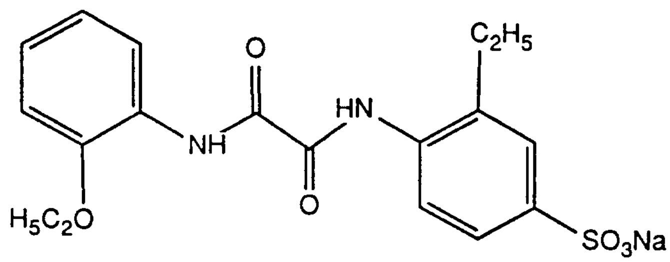 .

Die Herstellung des erfindungsgemässen asymmetrischen Oxalsäure-diarylamids der allgemeinen Formel (1) geschieht nach an sich bekannten Methoden, wie sie z.B. in der US-A-3,529,982 beschrieben sind. Man gelangt zu der Verbindung, indem man in an sich bekannter Weise Oxalsäure oder deren Ester in der ersten Stufe halbseitig durch Umsetzung von Oxalsäure oder Oxalsäureestern, insbesondere Alkylestern, mit etwa äquimolaren Mengen der entsprechenden Anilinverbindung amidiert. Eine bevorzugte Methode besteht zum Beispiel darin, dass Oxalsäure, Oxalsäurehalbester oder Oxalsäurediester mit gleichartigen oder verschiedenartigen Esterresten mit etwa äquimolaren Mengen der Anilinverbindung in der Schmelze oder in gegenüber den Reaktionspartnern inerten organischen Lösungsmitteln in Gegenwart von wasserfreier Borsäure bei Temperaturen zwischen etwa 50 und 200 °C kondensiert werden. Nach Isolierung des so erhaltenen Amid-Esters bzw. der Amid-Säure werden in der zweiten Stufe die noch verbliebene Carboxylgruppe bzw. Carboxylatgruppe des Oxalsäurehalbamids mit einem zweiten, von dem in der ersten Stufe verschiedenen Anilin unter analogen Bedingungen kondensiert, wobei im allgemeinen zweckmässig eine um 50 bis 100 °C höhere Temperatur, die etwa zwischen 100 und 250 °C liegt, gewählt wird. Auch hier werden etwa äquimolare Mengen eingesetzt.

Geeignete inerte organische Lösungsmittel, wie vorstehend erwähnt, sind insbesondere solche mit einem oberhalb etwa 160 °C liegenden Siedepunkt, also zum Beispiel höhere Benzolkohlenwasserstoffe oder halogenierte Benzole wie die Di- oder Trichlorbenzole.

Die Einführung der zweiten Amidgruppe kann andererseits auch durch halbseitige Verseifung des in der ersten Stufe hergestellten Amid-Esters zur Amid-Säure, Überführung in das Amid-Säurehalogenid und nachfolgende Amidierung der Säurehalogenidgruppe erfolgen.

Das so erhaltene Oxylsäurediarylamid, das noch freie Hydroxygruppen enthält, wird anschliessend in bekannter Weise verethert.

Das erfindungsgemässe asymmetrische Oxalsäure-diarylamid findet Verwendung zur photochemischen und thermischen Stabilisierung von natürlichen und synthetischen Polyamidfasermaterialien und deren Färbungen. In seiner Anwendung zeichnet es sich durch eine hohe Lichtstabilität und eine gute Faseraffinität aus und verleiht den mit diesen Verbindungen behandelten Fasermaterialien eine verbesserte photochemische Stabilität. Bei den natürlichen Polyamidfasem, insbesondere bei Wolle, äussert sich die verbesserte Photostabilität in einer Stabilisierung des Fasergrundweisses.

Unter Polyamidfasermaterial wird dabei synthetisches Polyamid, wie z.B. Polyamid 6, Polyamid 66 oder auch Polyamid 12, aber auch Wolle und Seide verstanden. Neben den reinen Polyamidfasem kommen vor allem Fasermischungen, z.B. von Polyamid 6/Wolle oder aus Polyurethan und Polyamid in Betracht, so z.B. Trikotmaterial aus Polyamid/Polyurethan im Mischungsverhältnis 70:30. Ebenso kommen Fasermischungen aus Polypropylen und Polyamid in Frage. Grundsätzlich kann das reine oder gemischte Polyamidmaterial in verschiedenen Verarbeitungsformen vorliegen, wie z.B. als Faser, Garn, Gewebe, Gewirke oder Teppiche.

Vor allem Polyamidmaterial, auch in Mischungen mit Polyurethan oder Polypropylen, das Licht und Hitze ausgesetzt ist und z.B. als Autopolsterstoff, Teppich oder Badebekleidungsstoff vorliegt, eignet sich besonders für die Anwendung der erfindungsgemässen Verbindung.

Die Verbindung der Formel (1) wird erfindungsgemäss aus wässrigem Bad appliziert. Die Menge der zugesetzten Verbindung hängt vom Substrat und der gewünschten Stabilisierung ab. Im allgemeinen setzt man 0,01 bis 10 Gew.%, bevorzugt 0,1 bis 5 Gew.%, bezogen auf das Substrat, zu.

Die Applikation der erfindungsgemässen Verbindung kann vor oder nach dem Färben, oder vorzugsweise während des Färbens, nach einem Ausziehverfahren bei Flottenverhältnissen von 1:5 bis 1:500, vorzugsweise 1:10 bis 1:50 erfolgen. Zweckmässigerweise wird die Verbindung dem Färbebad zugesetzt.

Die erfindungsgemässe Verbindung kann aber auch kontinuierlich mittels Niedrigauftragssystemen oder Heissapplikatonssystemen appliziert werden.

Beim Kontinue-Verfahren beträgt der Flottenauftrag zweckmässig 30-400 Gew.-%, vorzugsweise 75-250 Gew.-%. Zur Fixierung der applizierten Farbstoffe und der erfindungsgemässen Verbindung wird das Fasermaterial einer Hitzebehandlung unterworfen. Der Fixierprozess kann auch nach der Kaltverweilmethode erfolgen.

Die Hitzebehandlung erfolgt vorzugsweise durch ein Dämpfverfahren unter Behandlung in einem Dämpfer mit gegebenenfalls überhitztem Dampf bei einer Temperatur von 98 bis 105 °C während z.B. 1 bis 7, vorzugsweise 1 bis 5 Minuten. Die Fixierung der Farbstoffe und der Verbindung der Formel (1) gemäss dem Kaltverweilverfahren kann durch Lagerung der imprägnierten und vorzugsweise aufgerollten Ware bei Raumtemperatur (15 bis 30 °C), z.B. während 3 bis 24 Stunden erfolgen, wobei die Kaltverweilzeit bekanntlich von der Art des applizierten Farbstoffes abhängig ist.

Nach Beendigung des Färbeprozesses bzw. der Fixierung werden die hergestellten Färbungen auf übliche Weise gespült und getrocknet.

Die Färbung erfolgt in üblicher Weise, z.B. mit Metallkomplex-, Anthrachinon- oder Azofarbstoffen und Mischungen dieser Farbstoffe. Als Metallkomplexfarbstoffe werden die bekannten Typen, insbesondere die 1:2-Chrom- oder 1:2-Kobaltkomplexe von Mono- oder Disazo- oder Azomethinfarbstoffen eingesetzt, die in der Literatur in grosser Zahl beschrieben sind. Neben diesen kommen natürlich auch Farbstoffe aus anderen Farbstoffklassen in Frage, wie z.B. Dispersions- oder auch Reaktivfarbstoffe.

Die folgenden Herstellungsvorschriften der neuen Verbindungen und Anwendungsbeispiele dienen der Veranschaulichung der Erfindung. Teile bedeuten Gewichtsteile und Prozent Gewichtsprozente. Die Prozentangaben betreffend die Zusätze der einzelnen Färbe- bzw. Behandlungsbäder beziehen sich, wenn nicht anders angegeben, auf das Fasermaterial.

Beispiel 1: In einer Suspension aus 4,9 g (14,3 mol) 2-Ethoxy-2'-hydroxy-oxalsäure-bis-anilid-Natriumsalz hergestellt durch Kristallisation von 2-Ethoxy-2'-hydroxy-bis-anilid in Natronlauge) und 200 ml Aceton

wird eine Lösung aus 1,75 g (14,3 mmol) 1,3-Propansulton und 50 ml Aceton gegeben. Nach einer Stunde Erwärmen unter Rückfluss und Abkühlen wird der entstandene Niederschlag abgesaugt und getrocknet. Man erhält 5,45 g der Verbindung der Formel

(101) 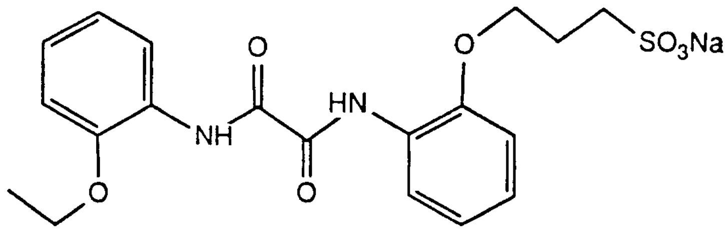 ,

welche aus Ethanol/Wasser (8:2) zu einer farblosen Substanz umkristallisiert wird.
Ausbeute 86%; $F_p$ 236-238 ° C.

| Elementaranalyse für $C_{19}H_{21}N_2O_7SNa \cdot 0{,}25\ H_2O$: | | | | |
|---|---|---|---|---|
| Gefunden: | 50,91% C; | 4,83% H; | 6,30% N; | 7,08% S |
| Berechnet: | 50,87% C; | 4,75% H; | 6,24% N; | 7,14% S |

Beispiel 2: Entsprechend Beispiel 1 werden 4,4 g (14,3 mmol) 2-Ethyl-2'-hydroxy-oxalsäure-bis-anilid-Natriumsalz mit 1,93 g (15,8 mmol) 1,3-Propansulton umgesetzt. Es werden nach Umkristallisieren aus 45%igem Ethanol 2,72 g der farblosen Verbindung der Formel

(102) 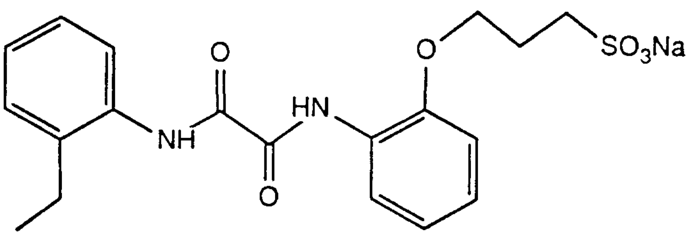

erhalten.
Ausbeute 44%; $F_p$ 210-212 ° C

| Elementaranalyse für $C_{19}H_{21}N_2O_6SNa \cdot 0{,}25\ H_2O$:: | | | | |
|---|---|---|---|---|
| Gefunden: | 52,78% C; | 4,98% H; | 6,58% N; | 7,12% S |
| Berechnet: | 52,71% C; | 5,00% H; | 6,47% N; | 7,40% S |

Beispiel 3: Zu einer Schmelze aus 9,48 g (40 mmol) 2-Ethoxy-oxalsäureanilidmonoethylester und 5,44 g Imidazol werden bei 100 ° C 8,02 g (38 mmol) 2-Ethylsulfanilsäure-Natriumsalz zugegeben. Es wird $\frac{1}{2}$ Stunde auf 110 ° C, anschliessend 2 Stunden auf 130 ° C erhitzt. Nach Abkühlen wird die Reaktionsmasse in 200 ml Wasser eingetragen. Der Niederschlag wird abgesaugt, mit 50 ml Eiswasser gewaschen und getrocknet. Man erhält 6,95 g der Verbindung der Formel

(103) 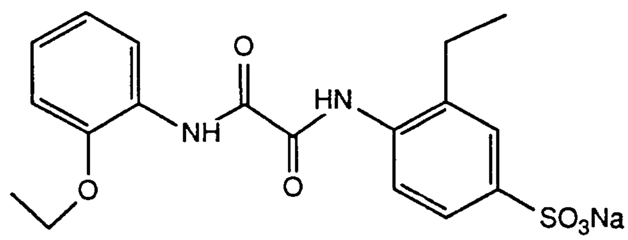 .

Ausbeute 44%; $F_p$ > 300 ° C

| Elementaranalyse für $C_{18}H_{19}N_2O_6SNa \cdot 0,25\ H_2O$: | | | | |
|---|---|---|---|---|
| Gefunden: | 51,6% C; | 4,7% H; | 6,8% N; | 7,5% S |
| Berechnet: | 51,60% C; | 4,69% H; | 6,68% N; | 7,65% S |

Beispiele 4 bis 29:

Herstellung der Verbindungen (104) bis (107), (110) bis (114), (117) bis (121) und (123) bis (129)

Analog zu Beispiel 2 werden zu einer Schmelze aus 40 mmol substituiertem Oxalsäureanilidmonoalkylester und 80 bis 200 mmol Imidazol bei 100 ° C 38 mmol gegebenenfalls substituierter Sulfanil- bzw. Metanilsäure zugegenen. Es wird ½ Stunde auf 110 ° C, anschliesssend 1 bis 3 Stunden auf 130 ° C erhitzt. Die Vollständigkeit des Umsatzes wird mittels Dünnschichtchromatographie überprüft. Nach Abkühlen wird die Reaktionsmasse in ca. 200 ml Wasser eingetragen. Der Niederschlag wird abgesaugt, mit Wasser gewaschen und getrocknet. Im Falle der Verbindungen (104) und (105) wird anstelle des Wassers Aceton, im Falle der Verbindungen (106), (107) und (114) Ethanol zur Aufarbeitung verwendet. Die Ausbeuten sind Tabelle I zu entnehmen.

Herstellung der Verbindung (116):

Zu einer Lösung aus 4,51 g (14,25 mmol) 2,5-Dimethoxy-4'-hydroxy-oxalsäure-bis-anilid und 100 ml Dimethylformamid werden 4,2 g (21,4 mmol) 30%ige methanolische Lösung von Natriummethylat sowie 4,42 g (21,4 mmol) 3-chlor-2-hydroxypropansulfonsäure-Natriumsalz gegeben. Nach 15-stündigem Rühren bei 150 ° C wird der entstandene Niederschlag (NaCl) abfiltriert und das Filtrat bei 75 ° C/0,13 Pa eingedampft. Der Rückstand wird in Wasser aufgenommen. Nach Zugabe Dimethylformamid/Ethanol umkristallisiert wird. Es werden 3,8 g eines weissen Pulvers erhalten.

Herstellung der Verbindung (109)

Die Verbindungen werden analog Verbindung (116) durch Umsetzung von 2-Ethoxy-2'-hydroxy-oxalsäure-bis-anilid hergestellt.

Herstellung der Verbindungen (108), (115) und (122)

Die Verbindungen werden analog Beispiel 1 hergestellt.

Herstellung der Ausgangsverbindung für die Verbindungen (115) und (116): 2,5-Dimethoxy-4'-hydroxy-oxalsäure-bisanilid

5,07 g (20 mmol) 2,5-Dimethoxy-oxalsäureanilid-monomethylester und 2g (18 mmol) 4-Aminophenol werden in Gegenwart katalytischer Mengen Bortrifluorid unter leichtem Vakuum auf 150 ° C erwärmt und entstehender Alkohol abdestilliert. Nach 5,5 Stunden wird abgekühlt und die Reaktionsmasse mit 40 ml Ethanol versetzt. Durch Kristallisation bei -5 ° C werden 3,4 g Rohprodukt erhalten, welches durch Waschen mit heissem Trichlorethylen gereinigt wird.
Fp. 204-205 ° C

| Elementaranalyse für $C_{16}H_{16}N_2O_5$: | | | |
|---|---|---|---|
| Gefunden: | 60,58% C; | 5,19% H; | 8,88% N; |
| Berechnet: | 60,75% C; | 5,1% H; | 8,86% N; |

Herstellung der Ausgangsverbindung für die Verbindung (122): 2-Methoxy-5-methyl-4'-hydroxy-oxalsäurebisanilid

8,3 g (41,5 mmol) 4-Hydroxy-oxalsäureanilid-monoethylester und 6,85 g (50 mmol) 2-Methoxy-5-methylanilin werden unter leichtem Vakuum auf 130 °C erwärmt und entstehender Alkohol abdestilliert. Nach 7 Stunden wird abgekühlt und die Reaktionsmasse mit 100 ml Aceton verrührt. Unlösliches Nebenprodukt wird abfiltriert und das Filtrat in 130 ml Wasser gegeben, wobei das Produkt ausfällt.
Ausbeute 6,27 g
Fp. 189-190 °C

| Elementaranalyse für $C_{16}H_{16}N_2O_4$: | | | |
|---|---|---|---|
| Gefunden: | 64,0% C; | 5,4% H; | 9,4% N; |
| Berechnet: | 63,99% C; | 5,3% H; | 9,32% N; |

Tabelle I:

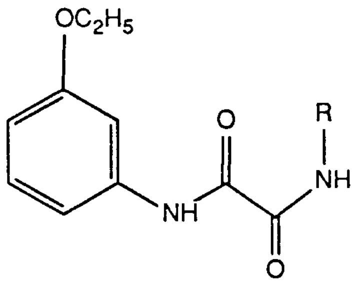

| Verbindung Nr. | R | Ausbeute [%] | Elementaranalyse |
|---|---|---|---|
| (104) | 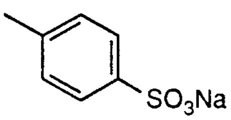 | 53 | $C_{16}H_{15}N_2O_6SNa$<br>gef.: 49,68% C; 4,11% H; 7,28% N; 7,84% S<br>ber.: 49,74% C; 3,91% H; 7,25% N; 8,3% S |
| (105) | 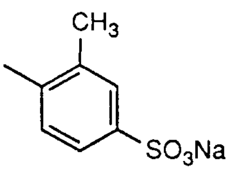 | 62 | $C_{17}H_{17}N_2O_6SNa$<br>gef.: 50,99% C; 4,14% H; 7,16% N; 7,90% S<br>ber.: 51,00% C; 4,28% H; 7,00% N; 8,01% S |
| (106) | 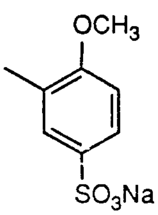 | 81 | $C_{17}H_{17}N_2O_7SNa$<br>gef.: 47,72% C; 4,32% H; 6,69% N; 7,40% S<br>ber.: 47,66% C; 4,31% H; 6,54% N; 7,48% S |

| Verbindung Nr. | R | Ausbeute [%] | Elementaranalyse |
|---|---|---|---|
| (107) | 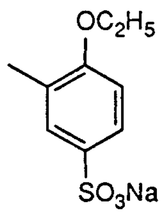 | 71 | $C_{18}H_{19}N_2O_7SNa \cdot \frac{1}{4}H_2O$<br>gef.: 49,39% C; 4,52% H; 6,77% N; 7,35% S<br>ber.: 49,70% C; 4,51% H; 6,44% N; 7,37% S |
| (108) | 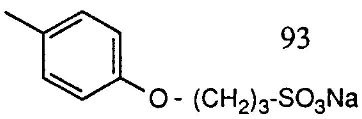 | 93 | $C_{19}H_{21}N_2O_7SNa$<br>gef.: 51,01% C; 4,82% H; 6,35% N; 7,09% S<br>ber.: 51,35% C; 4,76% H; 6,30% N; 7,21% S |
| (109) | 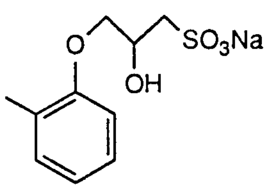 | 37 | $C_{19}H_{21}N_2O_8SNa \cdot H_2O$<br>gef.: 47,70% C; 4,70% H; 6,00% N; 7,20% S<br>ber.: 47,69% C; 4,84% H; 5,89% N; 6,70% S |

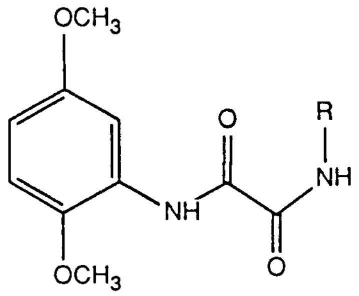

| Verbindung Nr. | R | Ausbeute [%] | Elementaranalyse |
|---|---|---|---|
| (110) | 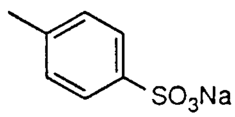 | 44 | $C_{16}H_{15}N_2O_7SNa$<br>gef.: 47,48% C; 3,82% H; 6,95% N; 7,86% S<br>ber.: 47,76% C; 3,76% H; 6,96% N; 7,97% S |
| (111) | 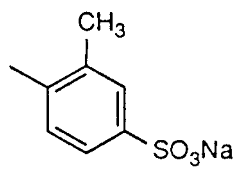 | 52 | $C_{17}H_{17}N_2O_7SNa \cdot \frac{1}{4}H_2O$<br>gef.: 48,40% C; 4,10% H; 6,80% N; 7,40% S<br>ber.: 48,51% C; 4,19% H; 6,65% N; 7,61% S |
| (112) | 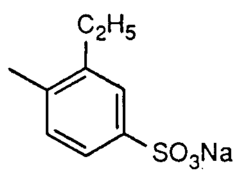 | 35 | $C_{18}H_{19}N_2O_6SNa \cdot \frac{1}{4}H_2O$<br>gef.: 51,60% C; 4,70% H; 6,80% N; 7,50% S<br>ber.: 51,60% C; 4,69% H; 6,68% N; 7,65% S |

| Verbindung Nr. | R | Ausbeute [%] | Elementaranalyse |
|---|---|---|---|
| (113) | 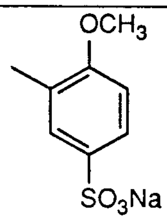 | 69 | $C_{17}H_{17}N_2O_8SNa$<br>gef.: 47,02% C; 4,06% H; 6,86% N; 7,05% S<br>ber.: 47,22% C; 3,96% H; 6,47% N; 7,41% S |
| (114) | 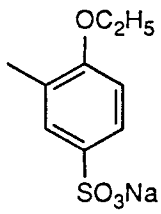 | 87 | $C_{18}H_{19}N_2O_8SNa$<br>gef.: 48,00% C; 4,30% H; 6,60% N; 6,80% S<br>ber.: 48,43% C; 4,29% H; 6,28% N; 7,18% S |
| (115) | 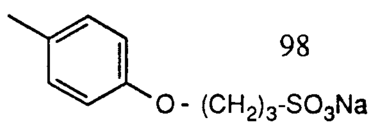 | 98 | $C_{19}H_{21}N_2O_8SNa \cdot \frac{1}{2}H_2O$<br>gef.: 48,53% C; 4,50% H; 5,98% N; 6,79% S<br>ber.: 48,61% C; 4,72% H; 5,96% N; 6,82% S |
| (116) | 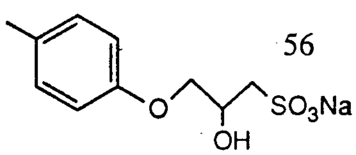 | 56 | $C_{19}H_{21}N_2O_9SNa$<br>gef.: 47,87% C; 4,64% H; 6,02% N; 6,64% S<br>ber.: 47,90% C; 4,44% H; 5,88% N; 6,73% S |

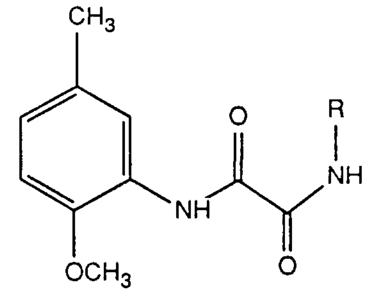

| Verbindung Nr. | R | Ausbeute [%] | Elemamtaranalyse |
|---|---|---|---|
| (117) | 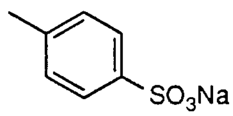 | 40 | $C_{16}H_{15}N_2O_6SNa$<br>gef.: 49,40% C; 4,00% H; 7,30% N; 8,30% S<br>ber.: 49,74% C; 3,91% H; 7,25% N; 8,29% S |
| (118) | 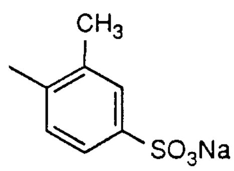 | 62 | $C_{17}H_{17}N_2O_6SNa \cdot \frac{1}{3}H_2O$<br>gef.: 50,20% C; 4,40% H; 7,10% N; 8,00% S<br>ber.: 50,20% C; 4,38% H; 6,89% N; 7,89% S |
| (119) | 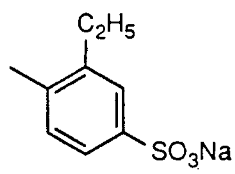 | 35 | $C_{18}H_{19}N_2O_6SNa \cdot \frac{1}{4}H_2O$<br>gef.: 51,76% C; 4,72% H; 6,82% N; 7,65% S<br>ber.: 51,60% C; 4,69% H; 6,68% N; 7,65% S |
| (120) | 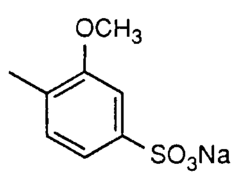 | 38 | $C_{17}H_{17}N_2O_7SNa \cdot 2\frac{3}{4}H_2O$<br>gef.: 46,57% C; 4,59% H; 6,59% N; 7,30% S<br>ber.: 46,52% C; 4,48% H; 6,38% N; 7,30% S |

| Verbindung Nr. | R | Ausbeute [%] | Elemamtaranalyse |
|---|---|---|---|
| (121) | 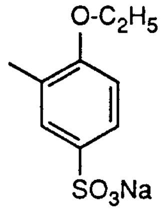 | 68 | $C_{18}H_{19}N_2O_7SNa\cdot\frac{1}{2}H_2O$<br>gef.: 49,27% C; 4,60% H; 6,49% N; 7,34% S<br>ber.: 49,20% C; 4,59% H; 6,37% N; 7,29% S |
| (122) | 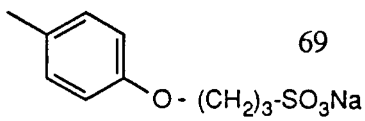 | 69 | $C_{19}H_{21}N_2O_7SNa\ \frac{1}{2}H_2O$<br>gef.: 51,00% C; 4,80% H; 6,30% N; 7,20% S<br>ber.: 51,35% C; 4,76% H; 6,30% N; 7,21% S |

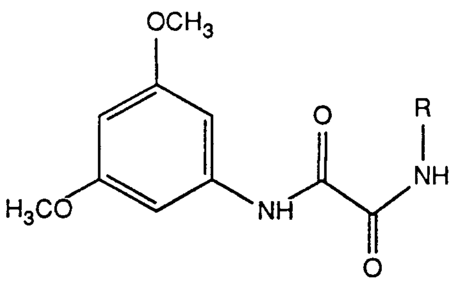

| Verbindung Nr. | R | Ausbeute [%] | Elementaranalyse |
|---|---|---|---|
| (123) | 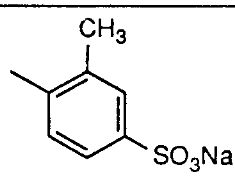 | 58 | $C_{17}H_{17}N_2O_7SNa$<br>gef.: 48,70% C; 4,10% H; 6,80% N; 7,60% S<br>ber.: 49,00% C; 4,12% H; 6,73% N; 7,70% S |

| Verbindung Nr. | R | Ausbeute [%] | Elementaranalyse |
|---|---|---|---|
| (124) | 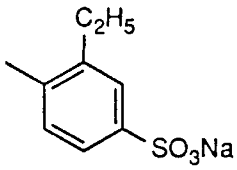 | 46 | $C_{18}H_{19}N_2O_7SNa$ |
| | | | gef.: 50,20% C; 4,40% H; 6,60% N; 7,50% S |
| | | | ber.: 50,23% C; 4,45% H; 6,51% N; 7,45% S |
| (125) | 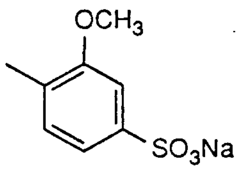 | 75 | $C_{17}H_{17}N_2O_8SNa \cdot \frac{1}{4} H_2O$ |
| | | | gef.: 46,63% C; 4,06% H; 6,55% N; 7,14% S |
| | | | ber.: 46,73% C; 4,03% H; 6,41% N; 7,33% S |
| (126) | 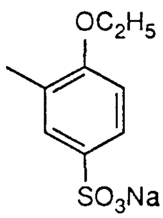 | 40 | $C_{18}H_{19}N_2O_8SNa$ |
| | | | gef.: 48,29% C; 4,42% H; 6,41% N; 7,06% S |
| | | | ber.: 48,43% C; 4,29% H; 6,28% N; 7,18% S |

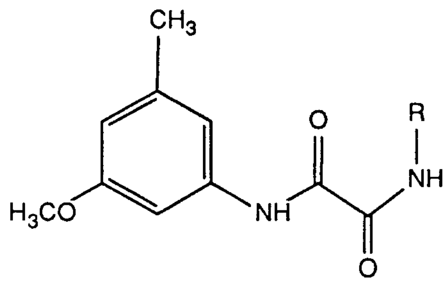

| Verbindung Nr. | R | Ausbeute [%] | Elementaranalyse |
|---|---|---|---|
| (127) | 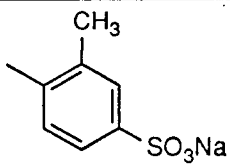 | 43 | $C_{17}H_{17}N_2O_6SNa$<br>gef.: 50,80% C; 4,4% H; 7,2% N; 8,20% S<br>ber.: 51,00% C; 4,28% H; 7,00% N; 8,01% S |
| (128) | 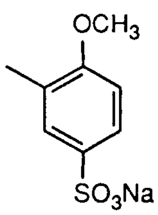 | 55 | $C_{17}H_{17}N_2O_7SNa$<br>gef.: 49,10% C; 4,20% H; 7,00% N; 7,70% S<br>ber.: 49,04% C; 4,12% H; 6,73% N; 7,70% S |
| (129) | 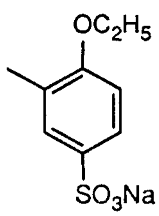 | 73 | $C_{18}H_{19}N_2O_7SNa \cdot \frac{1}{4} H_2O$<br>gef.: 49,60% C; 4,52% H; 6,54% N; 7,32% S<br>ber.: 49,71% C; 4,40% H; 6,44% N; 7,37% S |

Anwendungsbeispiele:

Beispiel 30: In einem ®AHIBA-Färbeapparat werden bei einem Flottenverhältnis von 1:25 2 Muster von je 10 g einer PA 6-Maschenware gefärbt. Beide Färbebäder enthalten jeweils folgende Zusätze: 0,5g/l Mono-Na-phosphat, 1,5 g/l Di-Na-phosphat sowie die Farbstoffe der Formeln (I) und (II). Alle Zusätze werden vor Zugabe gelöst.

(I)
0,04%

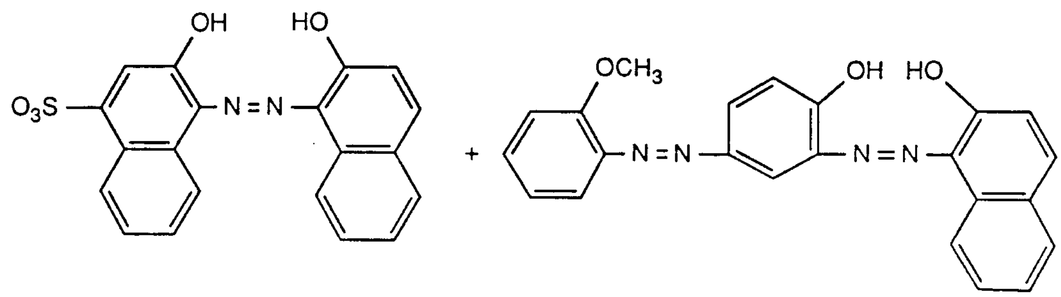

1:2 -Cr-Komplex; 81 Teile

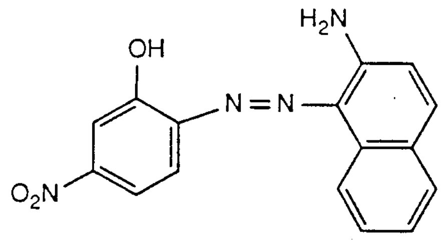

1:2 -Co-Komplex; 12 Teile
(Rest von 7 Teilen sind Salze und Tenside)

(II)
0,002%

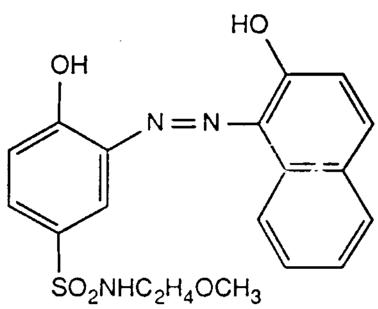

1:2 Co-Komplex

Während die Flotte 1 keine weiteren Zusätze enthält, wird der Flotte 2 noch 1% der Verbindung der Formel (101), in Wasser gelöst, zugesetzt.

Die so vorbereiteten Flotten erwärmt man auf 40 °C, geht mit dem Textilmaterial ein, behandelt bei dieser Temperatur 10 Minuten und erwärmt mit 2 °C/Minute auf 95 °C. Nach einer Färbezeit von 20 Minuten bei 95 °C werden 2% Essigsäure (80%ig) zugesetzt und 25 Minuten weitergefärbt. Danach kühlt man auf 60 °C ab, spült mit kaltem Wasser, zentrifugiert und trocknet bei 120 °C während 2 Minuten.

Die Färbungen werden auf ihre Heisslichtechtheiten nach DIN 75.202 (FAKRA) geprüft. Zur Ermittlung der photochemischen Stabilität werden die gefärbten Muster von 12x4,5 cm auf Karton aufgezogen und während 216 Stunden ( = 3 FAKRA-Cyclen) belichtet. Die Ergebnisse sind in Tabelle 2 aufgeführt.

Tabelle 2:

| Zusatz zum Färbebad | Lichtechtheit 144 h FAKRA | Reissfestigkeit/Dehnung [%] nach Belichtung 216 h FAKRA | |
|---|---|---|---|
| Kein Zusatz | 1H* | 12,3 | 33,3 |
| + 1% der Verbindung der Formel (101) | 2 | 42,8 | 53,7 |

* Muster hat nur noch geringe Reissfestigkeit

Es ist ersichtlich, dass durch den UV-Absorber Färbungen mit deutlich verbesserten Eigenschaften erzielt werden.

Beispiel 31: Man stellt, wie in Beispiel 30 beschrieben, je 3 Flotten ohne Farbstoff ( = Blindfärbungen her).

Flotte 1 enthält keine weiteren Zusätze,

Flotte 2 1% der Verbindung der Formel

(126)

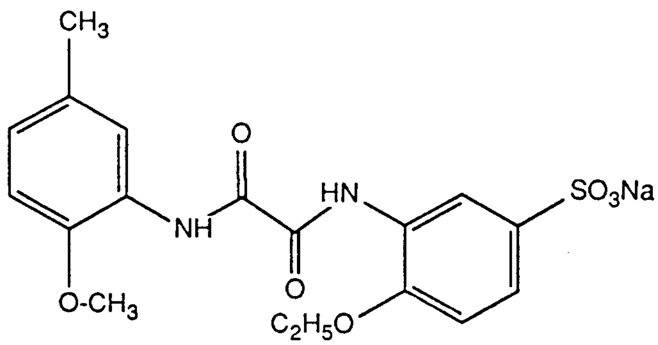

und

Flotte 3 1% der Verbindung der Formel

(113)

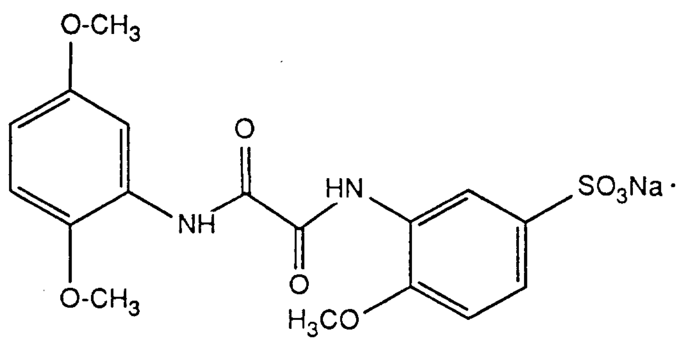

Das vorbereitete Trikotmaterial wird, wie in Beispiel 30 angegeben, behandelt, sodann nach FAKRA 216 h belichtet und schliesslich auf seine Stabilität nach SN 198.461 geprüft. Die Ergebnisse sind aus Tabelle 3 zu entnehmen:

Tabelle 3:

| Zusatz zum Färbebad | Reissfestigkeit/Dehnung [%] nach Belichtung 216 h FAKRA | |
|---|---|---|
| Flotte 1: Kein Zusatz | 4,3 | 17,1 |
| Flotte 2: + 1% der Verbindung der Formel (126) | 24,1 | 39,6 |
| Flotte 3: + 1% der Verbindung der Formel (113) | 27,7 | 40,7 |

Beispiel 32: Man bereitet 4 Muster eines PA 66-Trikots vor und setzt eine Färbeflotte wie in Beispiel 30 beschrieben an, mit dem Unterschied, dass

Flotte 1 keinen weiteren Zusatz enthält (=Blindfärbung),

Flotte 2 1% der Verbindung der Formel

(107) 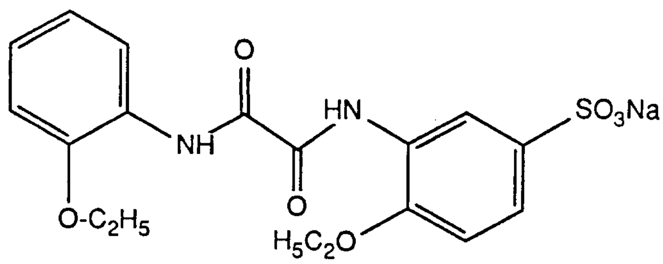

Flotte 3 1% der Verbindung der Formel

(114) 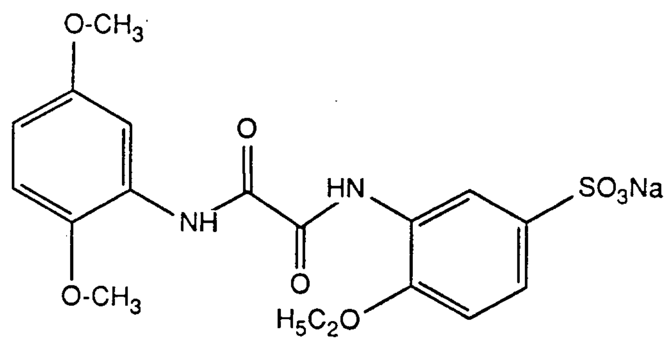

und

Flotte 4 1% der Verbindung der Formel

(115)

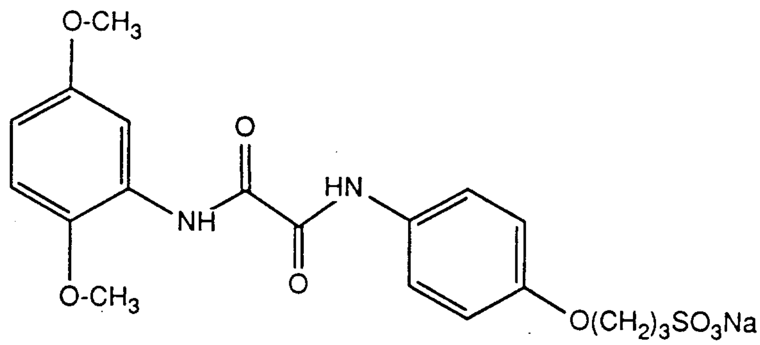

Es wird gefärbt und getestet wie in Beispiel 30 angegeben. Die Ergebnisse sind aus Tabelle 4 zu entnehmen:

Tabelle 4:

| Zusatz zum Färbebad | Lichtechtheit 144 h FAKRA | Reissfestigkeit/Dehnung [%] nach Belichtung 216 h FAKRA | |
|---|---|---|---|
| Flotte 1: Kein Zusatz * | << 1H | 4,3 | 16,8 |
| Flotte 2: + 1% der Verbindung der Formel (107) | 1-2 H | 16,9 | 30,6 |
| Flotte 3: + 1% der Verbindung der Formel (114) | 1-2 H | 35,4 | 40,9 |
| Flotte 4: + 1% der Verbindung der Formel (115) | 1-2 H | 31,5 | 39,5 |

* Material zerfällt

## Patentansprüche

**1.** Wasserlösliches, asymmetrisches Oxalsäurediarylamid der allgemeinen Formel

(1)

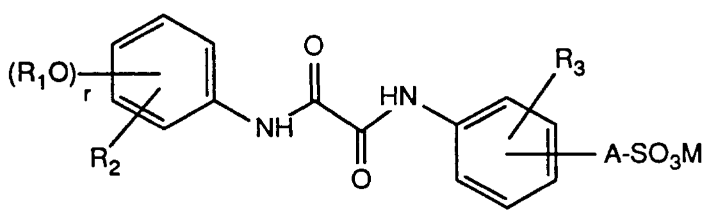

,

worin

- $R_1$ unabhängig voneinander unsubstituiertes oder durch Hydroxy oder Alkoxy substituiertes $C_1$-$C_5$Alkyl, unsubstituiertes oder durch $C_1$-$C_5$Alkyl substituiertes Benzyl
- $R_2$ Wasserstoff, Halogen, $C_1$-$C_{12}$Alkyl oder Phenyl$C_1$-$C_5$Alkyl
- $R_3$ Wasserstoff, Halogen, $C_1$-$C_{12}$Alkyl, Phenyl$C_1$-$C_5$Alkyl oder $C_1$-$C_5$Alkoxy
- A die direkte Bindung oder einen zweiwertigen Rest der Formel -O-Q-, worin
- Q unsubstituiertes oder mit Hydroxy substituiertes $C_1$-$C_6$Alkylen,
- M Wasserstoff oder Alkalimetall und

r 2,1 oder 0 bedeuten.

**2.** Oxalsäurediarylamid gemäss Anspruch 1, worin
Q Trimethylen oder

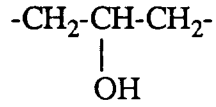

bedeutet.

**3.** Oxalsäurediarylamid gemäss Anspruch 1, das der Formel

(2) 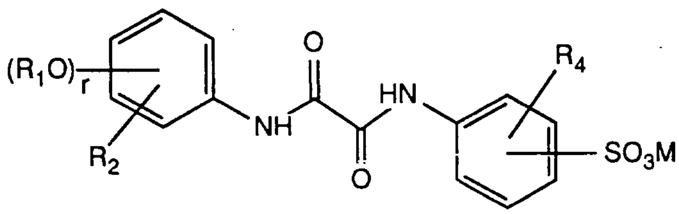

entspricht, worin

| | |
|---|---|
| $R_4$ | $C_1$-$C_{12}$Alkyl oder $C_1$-$C_5$alkoxy bedeutet und |
| $R_1$, $R_2$, M und r | die in Formel 1 angegebene Bedeutung haben. |

**4.** Oxalsäurediarylamid gemäss einem der Ansprüche 1 bis 3, das der Formel

(3) 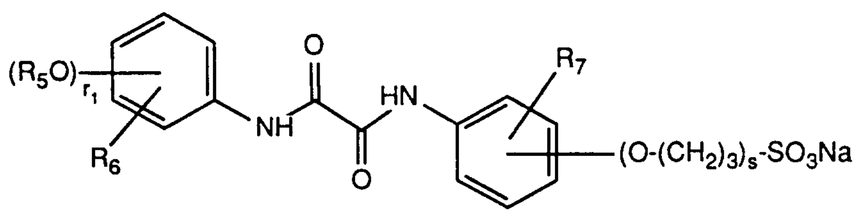 ,

entspricht, worin

| | |
|---|---|
| $R_5$ | $C_1$-$C_3$Alkyl, |
| $R_6$ | Wasserstoff, $C_1$-$C_3$Alkyl oder $C_1$-$C_3$Alkoxy, |
| $R_7$ | Wasserstoff oder $C_1$-$C_3$Alkoxy |
| $r_1$ | 0 oder 1, und |
| s | 0 oder 1 bedeuten. |

**5.** Oxalsäurediarylamid gemäss Anspruch 4, worin

| | |
|---|---|
| $R_5$ | Ethyl und |
| $R_6$ | Wasserstoff oder Ethyl bedeuten. |

**6.** Oxalsäurediarylamid gemäss Anspruch 5, das der Formel

(4) 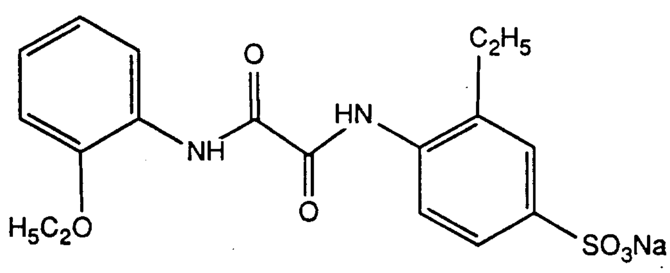

entspricht.

7. Verwendung eines Oxalsäurediarylamids gemäss einem der Ansprüche 1 bis 6 zur photochemischen und thermischen Stabilisierung von Polyamidfasermaterialien und deren Färbungen.

8. Das mit einem Oxalsäurediarylamid gemäss einem der Ansprüche 1 bis 7 behandelte Fasermaterial.

**Claims**

1. A water-soluble, asymmetrical oxalic acid diarylamide of general formula

(1) 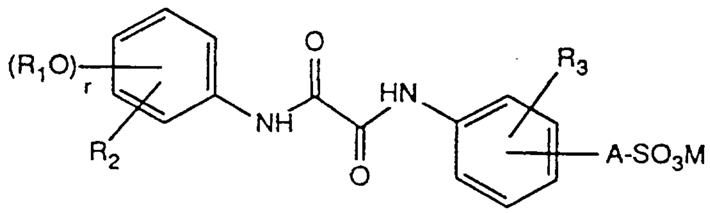 ,

wherein the substituents

| | |
|---|---|
| $R_1$ | are each independently of the other unsubstituted or hydroxyor alkoxy-substituted $C_1$-$C_5$alkyl or unsubstituted or $C_1$-$C_5$alkyl-substituted benzyl, |
| $R_2$ | is hydrogen, halogen, $C_1$-$C_{12}$alkyl or phenyl-$C_1$-$C_5$alkyl, |
| $R_3$ | is hydrogen, halogen, $C_1$-$C_{12}$alkyl, phenyl-$C_1$-$C_5$alkyl or $C_1$-$C_5$alkoxy, |
| A | is a direct bond or a divalent radical of formula -O-Q-, wherein |
| Q | is unsubstituted or hydroxy-substituted $C_1$-$C_6$alkylene, |
| M | is hydrogen or alkali metal, and |
| r | is 2, 1 or 0. |

2. An oxalic acid diarylamide according to claim 1, wherein Q is trimethylene or

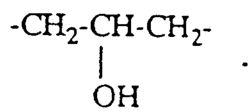 .

3. An oxalic acid diarylamide according to claim 1 of formula

(2) 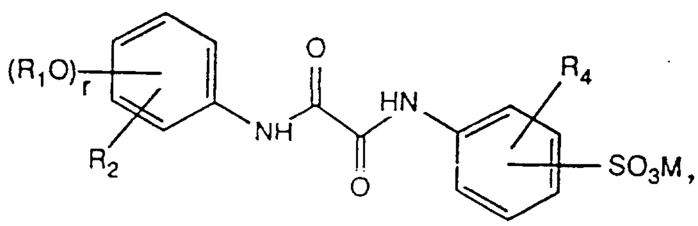

wherein

| | |
|---|---|
| $R_4$ | is $C_1$-$C_{12}$alkyl or $C_1$-$C_5$alkoxy, and |
| $R_1$, $R_2$, M and r | are as defined for formula 1. |

4. An oxalic acid diarylamide according to any one of claims 1 to 3 of formula

(3) 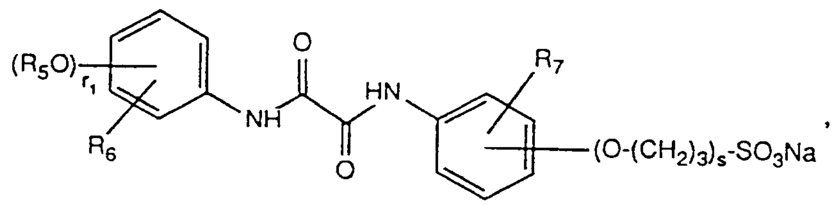

wherein

| | |
|---|---|
| $R_5$ | is $C_1$-$C_3$alkyl, |
| $R_6$ | is hydrogen, $C_1$-$C_3$alkyl or $C_1$-$C_3$alkoxy, |
| $R_7$ | is hydrogen or $C_1$-$C_3$alkoxy, |
| $r_1$ | is 0 or 1, and |
| s | is 0 or 1. |

**5.** An oxalic acid diarylamide according to claim 4, wherein

| | |
|---|---|
| $R_5$ | is ethyl and |
| $R_6$ | is hydrogen or ethyl. |

**6.** An oxalic acid diarylamide according to claim 5 of formula

(4) 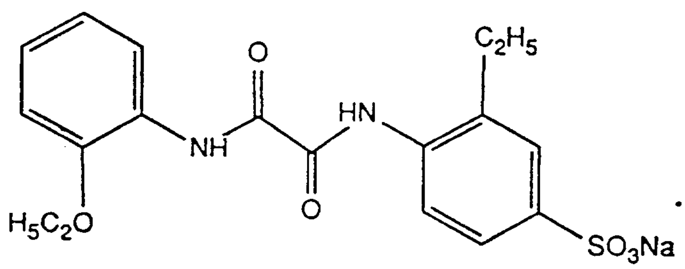

**7.** Use of an oxalic acid diarylamide according to any one of claims 1 to 6 for the photochemical and thermal stabilization of polyamide fibre materials and the dyeings produced thereon.

**8.** The fibre material treated with an oxalic acid diarylamide according to any one of claims 1 to 7.

**Revendications**

**1.** Diaryloxalamide asymétrique hydrosoluble de formule générale :

(1) 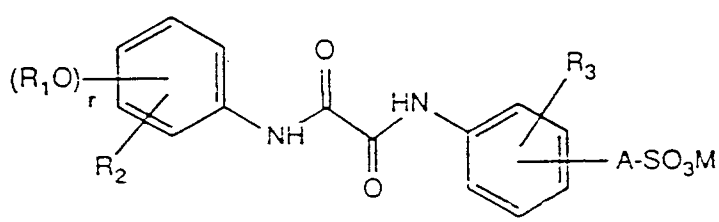

dans laquelle
chaque

| | |
|---|---|
| $R_1$ | représente indépendamment un résidu alkyle en $C_{1-5}$ non substitué ou substitué par un groupe hydroxy ou alcoxy, un résidu benzyle non substitué ou substitué par un groupe alkyle en $C_{1-5}$, |
| $R_2$ | représente un atome d'hydrogène, d'halogène, un groupe alkyle en $C_{1-12}$ ou phényle-(alkyle en $C_{1-5}$), |

R3 représente un atome d'hydrogène, d'halogène, un groupe alkyle en $C_{1-12}$ ou phényle-(alkyle en $C_{1-5}$) ou alcoxy en $C_{1-5}$,,

A représente une liaison directe ou un résidu divalent de formule -O-Q-, dans laquelle Q est un groupe alkylène en $C_{1-6}$ non substitué ou hydroxylé,

M représente un atome d'hydrogène ou un métal alcalin et

r est égal à 2, 1 ou 0.

**2.** Diaryloxalamide conforme à la revendication 1, dans lequel Q représente un résidu triméthylène ou $-CH_2-CH(OH)-CH_2-$.

**3.** Diaryloxalamide conforme à la revendication 1, qui correspond à la formule

(2)

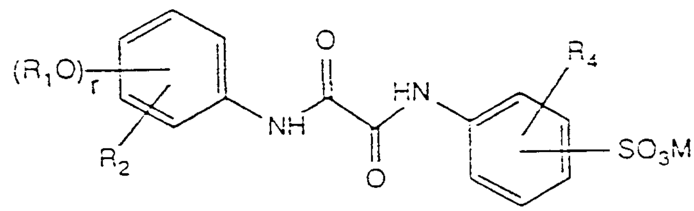

dans laquelle

| | |
|---|---|
| $R_4$ | est un groupe alkyle en $C_{1-12}$ ou alcoxy en $C_{1-5}$ et |
| $R_1$, $R_2$, M et r | ont la signification indiquée pour la formule (1). |

**4.** Diaryloxalamide conforme à une des revendications 1 à 3 correspondant à la formule

(3)

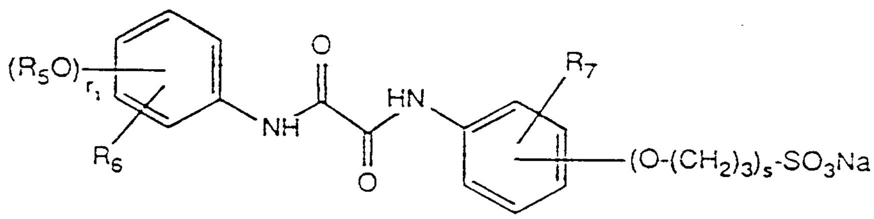

dans laquelle

$R_5$ est un groupe alkyle en $C_{1-3}$,

$R_6$ est un atome d'hydrogène ou un groupe alkyle en $C_{1-3}$,

$R_7$ est un atome d'hydrogène ou un groupe alcoxy en $C_{1-3}$,

$r_1$ est égal à 0, 1 ou 2 et

s est égal à 0 ou 1.

**5.** Diaryloxalamide conforme à la revendication 4, dans lequel

$R_5$ est un résidu éthyle et

$R_6$ un atome d'hydrogène ou un groupe éthyle.

**6.** Diaryloxalamide conforme à la revendication 5 correspondant à la formule

(4)

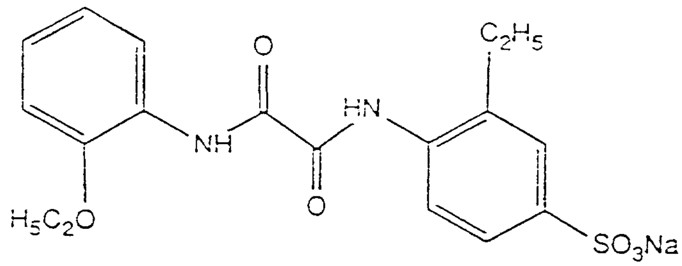

7. Utilisation d'un diaryloxalamide conforme à une des revendications 1 à 6 pour la stabilisation photochimique et thermique de fibres en polyamide et de leur teintures.

8. Matériau fibreux traité avec un diaryloxalamide conforme à une des revendications 1 à 7.